# DEMANDE DE BREVET EUROPEEN

(11) **EP 1 726 265 A1**
(43) Date de publication de la demande: **29.11.2006**
(21) Numéro de dépôt: 05447124.8
(22) Date de dépôt: 27.05.2005
(51) Int. Cl.: A61B 17/88, A61B 17/80

(54) **Méthode et équipement pour la simulation d'opération de chirurgie maxillo faciale et transfert de ce planning en salle d'opération**

(71) Demandeur: Université Catholique De Louvain, 1348 Louvain-La-Neuve (BE)
(72) Inventeur: Olszewski, Raphael, 1050 Bruxelles (BE); Tran Duy, Khanh, 1348 Louvain-La-Neuve (BE); Nicolas, Vincent, 5030 Gembloux (BE); Raucent, Benoit, 1348 Louvain-la-Neuve (BE); Reychler, Hervé, 1200 Bruxelles (BE)
(74) Mandataire: Brants, Johan P.E.

(57) **Abrégé**

La présente invention concerne un procédé de simulation médicale, caractérisé en ce qu'il comprend les étapes suivantes (a) l'affichage d'une image 3D du crâne d'un patient sur un écran, ledit crâne comprenant un passage de repère, (b) la mise en intercuspidation d'empreintes dentaires dudit patient après y avoir positionné le passage de repère décrit à l'étape (a), (c) la translation de la mise en intercuspidation de l'étape (b) à l'image 3 D , (d) l'analyse céphalométrique de l'image 3 D de l'étape (a), (e) l'établissement d'un plan de traitement et (f) la génération à partir de l'étape (e) de plaque d'ostéosynthése pré-pliée.

## Description

### Domaine de l'invention

La présente invention concerne la simulation médicale, en particulier la simulation d'opération de chirurgie maxillo-faciale telle que la chirurgie orthognathique et toute pathologie fracturaire humaine et non humaine. La présente invention concerne aussi des procédés et des équipements nécessaires pour la dite simulation.

### Etat de la technique

La chirurgie orthognathique consiste à corriger par diverses découpes osseuses, les malocclusions dentaires et les malpositions des maxillaires en permettant le déplacement et le repositionnement des fragments sectionnés (maximum 5 par maxillaire) dans les trois directions et orientations de l'espace. Les maxillaires (ou fragments de ceux-ci) sont ensuite refixés au moyen de plaquettes de fixation. Cette chirurgie s'applique à des patients, à l'issue de leur croissance, pour corriger des défauts survenus après des traumatismes, des malformations, ou plus rarement après des exérèses de tumeurs bénignes ou malignes.

Actuellement, les chirurgiens déterminent premièrement les déplacements nécessaires à l'obtention d'une harmonie faciale et d'une bonne fonction masticatoire au moyen d'une analyse bidimensionnelle (analyse de Delaire) effectuée sur une téléradiographie. Ces mesures ne permettent pas de déterminer précisément la position relative entre le maxillaire et la mandibule car celle-ci est régie par l'intercuspidation (engrènement des dents).

En conséquence, le repositionnement tridimensionnel peropératoire optimal, le contrôle des structures ostéotomisées, et le transfert des mesures précises obtenues sur le patient lors du diagnostic restent des éléments très aléatoires.

La précision lors de la simulation ainsi que le transfert correct vers le patient lors de l'opération, sont cependant garants du succès de l'intervention chirurgicale. En effet, le contrôle de l'occlusion dentaire et de la position des condyles mandibulaires est fondamental pour éviter la perte et/ou la dégradation progressive du résultat, ainsi que les dysfonctions musculaires et articulaires temporo-mandibulaires.

La simulation des interventions chirurgicales est actuellement réalisée de matière manuelle et artisanale sur des « articulateurs ». Les modèles en plâtre du patient sont sciés, selon des traits établis au crayon sur le plâtre, en tenant compte de lignes de référence. Les modèles sont ensuite déplacés manuellement puis stabilisés, généralement par de la cire. Les déplacements des fragments sont déterminés par l'analyse radiographique et celle des modèles, en se basant uniquement sur l'engrènement des dents selon les règles bien précises de l'occlusion dentaire, de manière statique et dynamique. Il en résulte une absence totale de reproductibilité de ces manoeuvres.

Durant cette étape, ce sont les mains de l'opérateur qui doivent maintenir en place ces fragments. La vérification des déplacements à effectuer est réalisée au moyen d'une latte
ou d'un compas sur le modèle en plâtre via des lignes de références tracées manuellement. La position relative des maxillaires à transposer sur le patient est ainsi obtenue après la simulation du mouvement de mâchoire via une articulation simplifiée.

Les déplacements ainsi estimés ne peuvent cependant pas être directement exécutés sur le patient durant l'opération. En effet, cela nécessiterait le contrôle des 6 degrés de liberté d'un maxillaire (ou de plusieurs fragments) avec une précision en deçà du millimètre (précision requise au niveau de l'intercuspidation) ainsi que la conservation de cette position durant la fixation. La solution à ce problème consiste à utiliser un splint ou clef occlusale qui, posé sur les dents du patient durant l'opération, permet la fixation des deux maxillaires dans la position relative souhaitée. Ce splint constitue donc le lien entre le diagnostic et la salle d'opération.

US 2003/0065259 décrit une méthode et un dispositif pour la fabrication de splint orthognathique. La méthode comprend la fabrication d'un splint physique final en associant deux techniques digitales un CT Scan 3D de la tête entière et un laser Scan 3D des arcades dentaires.

La principale difficulté rencontrée consiste dans le transfert précis et tridimensionnel de repères (lignes, angles et contours) déterminés sur des structures géométriques complexes (modèle en plâtre ou en résine) vers les tissus vivants, osseux principalement. Les conséquences principales de cette procédure sont une perte de temps dans la préparation des opérations pour les deux personnes nécessaires à sa réalisation, l'inadéquation entre les déplacements calculés lors du diagnostic et ceux imposés par le splint, l'absence de contrôle théorique sur les manipulations effectuées sur le patient. De plus, cette procédure "artisanale" ne permet pas d'envisager des déplacements trop complexes à réaliser, quelle que soit l'habilité et l'expérience du chirurgien.Durant l'opération, l'interposition du splint est précédée par la découpe d'un maxillaire (ou de fragments). Pour permettre les déplacements imposés par le splint une seconde découpe est parfois nécessaire. Celle-ci doit être réalisée avec précision (par rapport à la première découpe et, selon les déplacements désirés) afin de ne pas créer un « gap » qui empêcherait la calcification des os. Muni d'un compas, le chirurgien essaye par une série d'essais, d'obtenir de bons résultats. Le vissage des plaquettes de fixation intervient ensuite. Cette étape nécessite autant de patience que d'habileté afin de déformer les plaquettes de fixation à la forme du crâne et ainsi, stabiliser le mieux possible le résultat obtenu. Cette étape prend du temps et est très peu précise. Cette fixation engendre une inévitable déviation de la position souhaitée des os.

Lors d'une opération sur la mandibule, un problème supplémentaire survient lors de sa fixation à la partie condylienne. Si cette partie osseuse ne retrouve pas sa position initiale, le résultat postopératoire divergera du per-opératoire (et donc du diagnostic) et de nombreux troubles de la fonction masticatoire apparaîtront comme le TMD «Tempro Mandibular joint Disorders ».

La présente invention a donc pour objet de remédier aux inconvénients cités ci-dessus. En particulier la présente invention à pour objet de fournir un procédé de simulation de chirurgie permettant de ne plus passer par l'utilisation de splint en salle d'opération. Un autre objet de la présente invention est de fournir un procédé de simulation de chirurgie comprenant une intercuspidation dentaire réelle (contrôle visuel et tactile).

### Bref résumé de l'invention

La présente invention concerne donc dans un premier aspect, un procédé et l'équipement nécessaire pour une simulation médicale, comprenant (a) l'affichage d'une image 3D du crâne d'un patient sur un écran, ledit crâne comprenant un passage de repère, (b) la mise en intercuspidation d'empreintes dentaires dudit patient après y avoir positionné le passage de repère décrit à l'étape (a), (c) la translation de la mise en 'intercuspidation de l'étape (b) à l'image 3 D , (d) l'analyse céphalométrique de l'image 3 D de l'étape (a), (e) l'établissement d'un plan de traitement et (f) la génération à partir de l'étape (e) de plaque d'ostéosynthèse pré-pliée.

En particulier la présente invention concerne un procédé et l'équipement nécessaire pour une simulation d'opérations de chirurgie maxillo faciale (simulation de chirurgie maxillo-faciale) d'une part, et le transfert de cette simulation sur le patient, en salle d'opération d'autre part.

Le procédé selon l'invention comprend en premier lieu la reconstruction de l'image du crâne d'un patient, puis l' analyse céphalométrique 3D de la reconstruction (modèle virtuel), la manipulation d'empreinte dentaires du patient à l'aide d'un dispositif de déplacement et de mise en intercuspidation appelé ici « manipulateur » (cette étape peut se produire avant l'analyse céphalométrique), la fusion des informations entre l'analyse céphalométrique et la mise en intercuspidation sur manipulateur. Ce procédé peut comprendre aussi la simulation des découpes et des déplacements sur le modèle virtuel, et si nécessaire le repositionnement des condyles sur le modèle virtuel. Ce procédé comprend de plus une étape de transfert comprenant la conception de zone d'intérêt et le pré-pliage de plaque d'ostéosynthèse de tous types. La présente invention comprend aussi le transfert des traits d'ostéotomies, de l'orientation de direction des instruments chirurgicaux essentiels à l'opération (exemple : scie), les divers éléments de l'analyse céphalométrique 3D que sont les plans, points, droites, courbes, surfaces, volumes sur le patient pour guider le geste du chirurgien, et le transfert de la position des plaques d'ostéosynthèse sur le patient pour guider le geste du chirurgien.

En particulier, la présente invention concerne un procédé de simulation de chirurgie maxillo-faciale caractérisé en ce qu'il comprend les étapes suivantes :
i) le positionnement d'un passage de repère sur les dents d'un patient et le passage du patient dans un système d'imagerie 3D pour générer un modèle virtuel du crâne du patient,
ii) l'analyse céphalométrique 3D du modèle virtuel obtenu à l'étape (i),
iii) l'obtention d'empreintes dentaires dudit patient et leur positionnement dans un dispositif de déplacement manuel et de mise en intercuspidation desdites empreintes dentaires,
iv) le positionnement du passage de repère de l'étape (i) sur les empreintes dentaires et la mise en intercuspidation des empreintes à l'aide du dispositif de déplacement et de mise en intercuspidation,
v) la mesure des positions des empreintes après mise en intercuspidation et la transmission desdites positions sur le modèle virtuel généré à l'étape (i),
vi) la détermination sur le modèle virtuel d'une ou plusieurs zones de positionnement pour des plaques d'ostéosynthèses, et le pré-pliage virtuel desdites plaques.
   Selon un mode de réalisation particulier de l'invention, les étapes (ii) et (iii) se font en parallèle. Selon un autre mode de réalisation particulier de l'invention, l'étape (iii) a lieu avant l'étape (ii).

Selon un mode de réalisation particulier de l'invention, lorsqu'une position optimale d'intercuspidation est obtenue à l'étape (ii) la position est bloquée et le déplacement mesuré et l'impact affiché.

Selon un mode de réalisation particulier de l'invention, ledit procédé comprend en outre une étape de fabrication de zones de positionnement déterminées à l'étape (vi). De préférence ladite fabrication se fait par prototypage.

Selon un mode de réalisation particulier de l'invention, ledit procédé comprend une étape de pré-pliage de plaques d'ostéosynthèses à l'aide desdites zones de positionnement.

Selon un mode de réalisation particulier de l'invention, ledit procédé comprend après l'analyse céphalométrique une étape de tracé de traits d'ostéotomies virtuels.

Selon un mode de réalisation particulier de l'invention, le procédé selon l'invention comprend entre l'étape (a) et (b) ou (i) et (iii) une étape de repositionnement des condyles du patient sur le modèle virtuel.

Le procédé de simulation utilise un dispositif mécanique de déplacement et de mise en intercuspidation d'empreintes dentaires (le manipulateur), permettant de mesurer et de bloquer avec précision et automatiquement une position obtenue en déplacent manuellement un objet. La présente invention concerne aussi ce manipulateur ainsi que son utilisation dans le procédé de simulation selon l'invention.

Ce dispositif selon l'invention est adapté au déplacement manuel et à la mise en intercuspidation d'empreintes et est caractérisé en ce qu'il comprend : une base fixe sur laquelle sont connectées six jambes articulées, et l'extrémité supérieure de chacune des six jambes est connecté à une plateforme mobile.

Selon un mode particulier de réalisation de l'invention, chaque jambe du dispositif (manipulateur) comprend : (1) une articulation rotoïde à 1 degré de liberté en rotation qui lie un premier segment rigide à ladite base, (2) le premier segment rigide, (3) une articulation sphérique qui lie le premier segment à un second segment rigide, (4) un second segment rigide, et (5) une articulation sphérique qui lie le second segment rigide à ladite plate-forme. Selon un mode de réalisation particulier de l'invention l'articulation sphérique à 3 degrés de liberté en rotation. Selon un mode particulier de réalisation de l'invention l'articulation sphérique comprend une première bille positionnée sur un palier lisse à trois points de contact constitué chacun d'une bille secondaire de diamètre inférieur à la première bille, ledit palier comprenant un aimant dans un cylindre isolant positionné dans le palier. Selon un mode particulier de réalisation de l'invention ladite première bille, les billes secondaires et le palier sont en matériau magnétique.

Selon un mode particulier de réalisation de l'invention le dispositif comprend de plus un ou plusieurs freins électromagnétiques sans jeu liés à ladite base. Selon un mode particulier de réalisation de l'invention le dispositif comprend de plus un ou plusieurs capteurs de rotation positionnés sur lesdites articulations rotoïdes pour mesurer les variations de position. Selon un mode de réalisation particulier de l'invention le dispositif est connecté à un ordinateur avec une interface qui permet le calcul des positions du dispositif.

La présente invention concerne aussi une articulation sphérique particulièrement adaptée au manipulateur selon l'invention, caractérisée en ce qu'elle comprend une première bille positionnée sur un palier lisse à trois points de contact constitué chacun d'une bille secondaire de diamètre inférieur à la première bille, ledit palier comprenant un aimant dans un cylindre isolant positionné dans le palier. Selon un mode de réalisation particulier, ladite première bille, les billes secondaires et le palier sont en matériau magnétique.

La présente invention concerne aussi un dispositif d'articulation adapté à la simulation de chirurgie comprenant un passage de repère, un bâti et un manipulateur et concerne aussi une méthode de fabrication de passage de repère à l'aide dudit dispositif d'articulation.

Le procédé de simulation selon l'invention comprend aussi une étape de pré-pliage pour transférer une position relative désirée de structures osseuses d'une simulation vers un patient. La présence invention concerne aussi une méthode de transfert de position relative d'os sur un patient, conformément à une simulation via le pliage de plaque d'ostéosynthèse de tous type. La présente invention concerne aussi une méthode de pré-pliage de plaques d'ostéosynthèses comprenant une étape de prototypage des zones osseuses concernées, en position relative désirée où seront positionnées les plaques, pour effectuer ce pré-pliage. Cette méthode de pré-pliage se fait conformément à un état virtuel (software) désiré du patient, qui une fois positionnée sur le patient réel, impose la même position relative des os que celle désiré.

La présente invention concerne aussi un système pour la simulation de chirurgie maxillo-faciale comprenant : un passage de repère, un ordinateur comprenant un logiciel d'analyse céphalométrique 3D, un dispositif pour l'acquisition de données céphalométriques 3D et un manipulateur pour le déplacement et la mise en intercuspidation d'empreintes dentaires. Ce système permet à un opérateur de déterminer manuellement sur des modèles de dentition d'un patient, une position d'intercuspidation, de mesurer la variation de position (de manière continue) engendrée par ce déplacement manuel, de bloquer cette position et d'engendrer le même déplacement dans le logiciel de simulation. Ce système de simulation peut aussi comprendre un ordinateur avec un logiciel de simulation virtuelle 3D d'opération. Selon un mode de réalisation de l'invention ce système de simulation peut aussi comprendre un dispositif de prototypage de zone d'os telle qu'une micro-imprimante 3D, un ou plusieurs éléments physiques 3D (zones d'os) enrichis en information 3D provenant de la simulation, un guide au pliage préopératoire des plaques d'ostéosynthèse, et/ou un système de réalité augmentée.

La présente invention concerne aussi un dispositif de reconstruction (digitalisation) (par exemple par éclairage structuré) de l'occlusion ou des surfaces dentaires au moyen de mesure sur des mordus complets ou partiels.

La présente invention concerne également un dispositif de prédécoupe de structures osseuses selon une simulation pour remplir les gaps créés par les ostéotomies.

La présente invention concerne aussi un kit préopératoire comprenant : et au moins une zone d'os et au moins une plaque préformée adaptée à ladite zone d'os. Ce kit peut aussi comprendre un manuel d'utilisation comprenant des informations pour le guidage intra opératoire.

La présente invention concerne aussi un kit de simulation de chirurgie maxillo-faciale comprenant : un passage de repère comprenant une partie à usage unique et une partie à usage constant, un ordinateur avec une analyse céphalométrique 3D, et un appareil d'axiographie 4D (caméras digitales, marqueurs spécifiques à usage unique, outil de fixation endobuccal à usage unique), un ordinateur avec un logiciel de traitement des informations venant de l'axiographie 4D. Ce kit peut aussi comprendre un ordinateur avec un logiciel de simulation virtuelle 3D de préparation des opérations (chirurgie maxillo-faciale, chirurgie ORL, chirurgie orthopédique, neurochirurgie), et/ou un manipulateur à 6 degrés de liberté pour la mesure 3D de déplacement relatif des éléments physiques 3D et fabrication de splint (à usage unique). Ce kit peut aussi comprendre une micro imprimante 3D, une ou plusieurs zones d'os, un ou plusieurs guides au pliage préopératoire des plaques d'ostéosynthèse, et/ou des plaques d'ostéosynthèse (maxi plaques, inserts, tiges, etc.) pré pliées (à usage unique).

Ce kit peut aussi comprendre un ou plusieurs éléments suivants sélectionnés parmi (a) un ordinateur pour le guidage d'opération de chirurgie comprenant avec un système de réalité augmentée dans lequel ledit système de réalité augmentée comprend une indication visuelle des positions des trous à forer dans l'os une indication visuelle des endroits pour découper l'os, l'orientation de direction pour des instruments chirurgicaux essentiels à l'opération (e.g. scie), indication visuelle de divers éléments de l'analyse céphalométrique 3D que sont les plans, points, droites, courbes, surfaces, volumes, les zones dangereuses à éviter pendant l'opération par exemple : racines des dents, émergences de nerfs, vaisseaux; (b) une caméra digitale, (c) des marqueurs spécifiques placés sur les tissus du patient (à usage unique) et sur les instruments (selon le type de chirurgie), avec une partie à usage unique et une partie à usage constant, un écran, une paire de lunettes enrichis en informations 3D et 4D.

La présente invention concerne aussi des aides opératoires reproduisant fidèlement les mouvements déterminés lors du procédé de simulation. Ces aides opératoires consistent en un robot peropératoire, la fabrication d'un modèle RP (*rapid prototyping*) de la région à ostéosynthétiser, et le pré-pliage des plaques d'ostéosynthèses. La présente invention concerne aussi des outils de repérage peropératoire tels que des lunettes de réalité virtuelle augmentée; et des outils pour la prédiction du comportement/déformation des tissus mous sous l'effet de déplacement des tissus osseux sous-jacents.

Les procédés, systèmes et équipements selon la présente invention sont particulièrement adapté au transfert de planning de chirurgie des tissus durs en salle d'opération (utilisable sur tous les os et avec n'importe quel type de plaque (plastic, titane,...).

D'autres aspects, particularités et avantages de la présente invention apparaîtront à la lecture de la description qui va suivre et des exemples qui l'illustrent.

Brève description des figures :
La Figure 1 illustre deux exemples de passages de repères selon un mode de réalisation de l'invention.
La Figure 2 représente un schéma de principe du manipulateur passif selon un mode de réalisation particulier de l'invention. Les détails ne sont représentés que sur une jambe
La Figure 3 illustre un manipulateur selon un mode de réalisation particulier de l'invention.
La Figure 4 illustre la structure mécanique développée du manipulateur selon un mode de réalisation particulier de l'invention.
La Figure 5 illustre schématiquement une articulation sphérique selon un mode de réalisation particulier de l'invention.
La Figure 6 illustre schématiquement un exemple de bâti selon un mode particulier de l'invention.
Les Figures 7, 8 et 9 illustrent schématiquement un procédé de pré-pliage de plaques selon un mode de réalisation particulier de l'invention.
Les Figures 10 et 11 illustrent schématiquement des exemples de découpes classiques (Figure 10) ou complexes (Figure 11).
Les Figures 12, 13 et 14 représentent des diagrammes illustrant différentes étapes du procédé selon un mode de réalisation particulier de l'invention.
La Figure 15 représente une photo illustrant le positionnement d'un passage de repère sur un patient représenté par dans la photo par un crâne, selon un mode de réalisation de l'invention.
La Figure 16 représente une photo du manipulateur sur lequel les modèles et le passage de repère sont positionnés selon un mode de réalisation de l'invention, La Figure 17 représente une photo d'une zone fabriquée par prototypage selon un mode de réalisation de l'invention.
La Figure 18 représente une photo d'une zone fabriquée par prototypage comprenant les plaques préformées selon un mode de réalisation de l'invention.
La Figure 19 représente une photo illustrant une analyse de Delaire 2D.
La Figure 20 représente une photo illustrant une analyse de analyse ACRO 3D.
La Figure 21 représente une image illustrant le placement de point M droit et gauche sur reconstruction 3D.
La Figure 22 représente un plan C1 sur l'image de la Figure 25. Plan supérieur de la base du crâne.
La Figure 23 représente des plans C1, C2, C3, C4 sur l'image de la Figure 25. Plan vertical postérieur.
La Figure 24 représente une photo d'empreintes adaptées à une utilisation dans la présente invention.
La Figure 25 représente une photo montrant les empreintes en plâtre fixées sur le manipulateur.
La Figure 26 représente deux photos montrant le positionnement du repère sur les empreintes positionnées sur le manipulateur.
La Figure 27 représente une photo montrant l'opérateur manipuler le manipulateur et qui amène la mandibule en intercuspidation.

### Description détaillée de l'invention.

Dans un premier aspect, la présente invention concerne un procédé de simulation médicale, en particulier la simulation d'opération de chirurgie maxillo-faciale telle que la chirurgie orthognathique et toute pathologie fracturaire humaine et non humaine. Le procédé de simulation selon l'invention est très rapide et très précis. Il permet de fusionner les résultats pour les déplacements de l'intercuspidation et de l'analyse céphalométrique. Une simulation plus proche de la réalité est ainsi obtenue et une plus grande précision lors de la réalisation de l'opération sur le patient est obtenue.

Les termes « simulation préopératoire » ou « planning préopératoire » désignent dans la présente description et dans les revendications auxquelles elle donne lieu, l'ensemble des actions visant à préparer une opération à l'avance au moyen d'un poste de travail.

Le terme « analyse céphalométrique 3D » désigne dans la présente description et dans les revendications auxquelles elle donne lieu, le choix des points anatomiques sur un support radiologique 3D (par exemple CT Scan 3D, RMN 3D, échographie 3D, CAT Scan 3D, laser Scan 3D, holographie 3D, etc.) avec une reconstruction 3D de l'objet à analyser, suivi de mesures entre les points anatomiques, de mise en place de segments de droites, de droites, de plans, d'ensembles de plans, de surfaces, d'angles entre les plans, de volumes entre les plans,- de volumes indépendants des plans. Cette analyse peut comprendre en outre la comparaison des mesures du patient aux normes céphalométriques 3D ainsi que la recherche d'une harmonie cranio-maxillo-faciale 3D adaptée à chaque individu (à partir des lois générales) sous forme de plans de symétrie droite-gauche et de plan d'occlusion idéal individuel 3D. L'analyse céphalométrique 3D dans le contexte de la présente invention est à différencier de la prise de mesures entre des points anatomiques sur des coupes 2D acquises lors d'un CT Scan 3D. L'analyse céphalométrique 3D dans le contexte de la présente invention est à différencier du placement des points de référence anatomiques sur la reconstruction 3D CT et leur ré-projection sur des tomographies 2D.

Le terme « 2D » : signifie dans la présente description deux dimensions (x, y). Le terme « 3D » signifie dans la présente description trois dimensions (x, y, z). Le terme « 4D » signifie dans la présente description quatre dimensions (3D et temps).

Le terme « élément passage de repère» désigne dans la présente description et dans les revendications auxquelles elle donne lieu un dispositif qui corrèle la simulation virtuelle 3D et le manipulateur selon la présente invention. Ce dispositif peut aussi corréler la simulation virtuelle 3D, le manipulateur à 6 degrés de liberté et la situation peropératoire.

Le terme « manipulateur » désigne dans la présente description et dans les revendications auxquelles elle donne lieu, un dispositif de déplacement manuel et de mise en intercuspidation d'empreintes dentaires.

Les termes « modèle » « modèle dentaire » « empreinte » ou « empreinte dentaire » désigne l'empreinte en plâtre ou autre matériaux de l'arcade dentaire d'un patient.

Le terme « intercuspidation » signifie dans la présente description les contacts des cuspides des dents des 2 arcades dentaires antagonistes.

Le terme « ostéotomie » désigne la section d'un os, effectuée dans le but d'en modifier la direction, la longueur ou la position.

Les termes « zone d'intérêt » ou « zone d'os » désignent dans la présente description et dans les revendications auxquelles elle donne lieu un élément physique 3D, anatomique, représentant une partie d'os corrigée par simulation virtuelle. L'élément 3D corrigé peut porter des informations spatiales d'orientation, de taille et de forme pour les plaques à placer, ainsi que les informations sur les endroits dangereux à éviter lors du placement des plaques.

Les termes « pliage préopératoire » « pré-pliage »ou « préformage » des plaques désignent dans la présente description et dans les revendications auxquelles elle donne lieu la réalisation de pliage de plaque d'ostéosynthèse (plaques non pliées décrites dans l'art antérieur) avant l'opération proprement dite.

Le terme « transfert en salle d'opération »désigne dans la présente description et dans les revendications auxquelles elle donne lieu le kit pouvant être fourni au chirurgien pour réaliser son opération de manière plus précise et plus rapidement

Le terme « réalité augmentée » désigne dans la présente description et dans les revendications auxquelles elle donne lieu l'ajout des informations visuelles, auditives et/ou tactiles qui se surajoutent avec la réalité qui nous entoure. Ce terme comprend par exemple la surprojection -sur le champ opératoire, sur des écrans et /ou dans des lunettes sur les yeux du chirurgien, l'orientation de direction pour des instruments chirurgicaux essentiels à l'opération (scie), indication visuelle de divers éléments de l'analyse céphalométrique 3D que sont les plans, points, droites, courbes, surfaces, volumes, les zones dangereuses à éviter pendant l'opération (par exemple : racines des dents, émergences de nerfs, vaisseaux), etc. des lignes selon lesquelles il faut découper les os, inciser les tissus mous, positionner les implants, etc.

Le terme « Axiographie 4D»: désigne dans la présente description et dans les revendications auxquelles elle donne lieu un outil de mesure dynamique 3D du mouvement d'ouverture et de fermeture individuelle de la mandibule comprenant des marqueurs placés sur les tissus du patient et suivis par des caméras digitales en continu, avec intégration de l'imagerie RMN dynamique 4D de l'articulation temporo-mandibulaire et des mesures électrophysiologiques des muscles masticateurs de la face. La présente invention permet de personnaliser la dynamique de rotation condylienne par la mise en place de la méthode d'axiographie 4D.

Le procédé selon la présente invention, comprend l'étude tridimensionnelle des structures céphaliques, par exemple au départ d'un CT scan céphalique. Le procédé utilise un logiciel destiné à permettre une analyse céphalométrique tridimensionnelle. Ce logiciel permet une approche diagnostique « volumétrique » et quantifiable de manière précise et reproductible. L'analyse céphalométrique s'inscrit dans le plan de traitement virtuel réalisé à la console. Le transfert des données quantitatives de l'analyse céphalométrique vers la simulation des déplacements des pièces osseuses est rigoureusement objectif et précis.

Le procédé selon l'invention permet de simuler virtuellement tous les mouvements des fragments ostéotomisés (avec jusqu'à 66 degrés de liberté) et de robotiser le déplacement parfaitement contrôlé des fragments (en plâtre ou en résine). Le procédé de simulation selon l'invention permet en outre une mesure dynamique (en continu) du changement de position d'un modèle en plâtre par rapport à l'autre. Le procédé de simulation selon l'invention permet de scinder le modèle de l'arcade supérieure (et inférieure) en plusieurs fragments et de les déplacer de manière indépendante.

Pour la corrélation entre les modèles en plâtre sur le manipulateur et l'image virtuelle 3D un passage de repère est utilisé. Le passage de repère selon l'invention est un dispositif conçu pour l'application de chirurgie maxillo-facial et adapté au manipulateur selon l'invention.

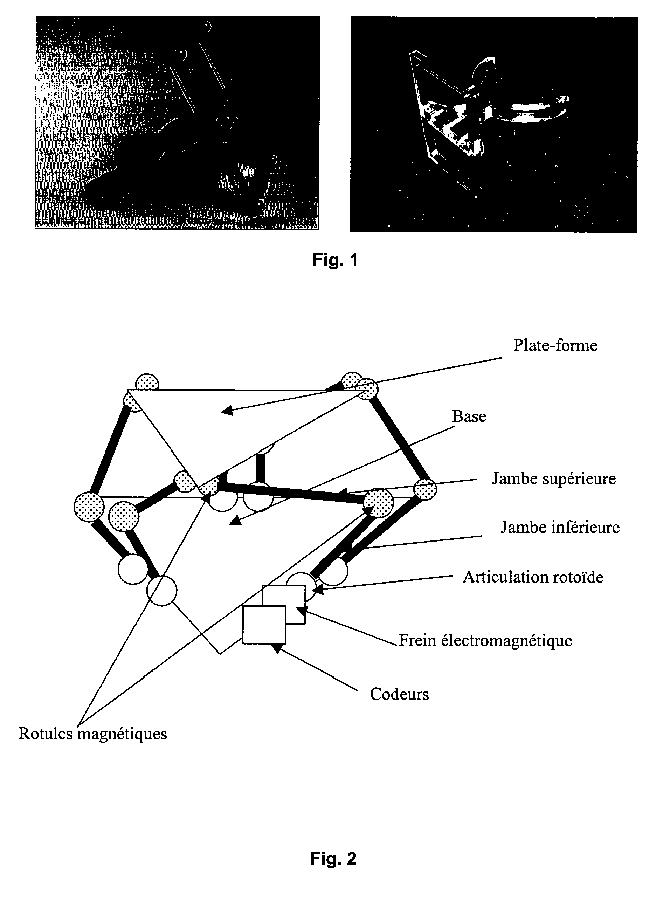

Le passage de repère est un dispositif comprenant un cadre par exemple en plexy sur lequel repose des billes, par exemple en métal, de préférence en titane. Le cadre est connecté à un support par exemple en aluminium sur lequel est fixé un mordu qui peut être en matériau identique à celui utilisé pour la fabrication de splints. Des exemples de passages de repères sont illustrés à la Figure 1. Selon un mode particulier de réalisation, le passage de repère comprend au moins 3 billes.

L'utilisation d'un tel passage de repère, visible dans un CT scan peut être utilisé par d'autres systèmes de mesures pour passer les repères entre applications. Ici le lien s'effectue entre trois systèmes :: le logiciel, le système de navigation optique et le système mécanique (le manipulateur) et permet de repositionner en même temps les empreintes dentaires dans une position relative identique. Le passage de repère est adapté pour son positionnement sur le manipulateur de la présente invention.

Selon un mode de réalisation le passage de repère selon l'invention peut être fabriqué non pas sur le patient mais sur un modèle dentaire de telle sorte que celui-ci comporte plus d'information.

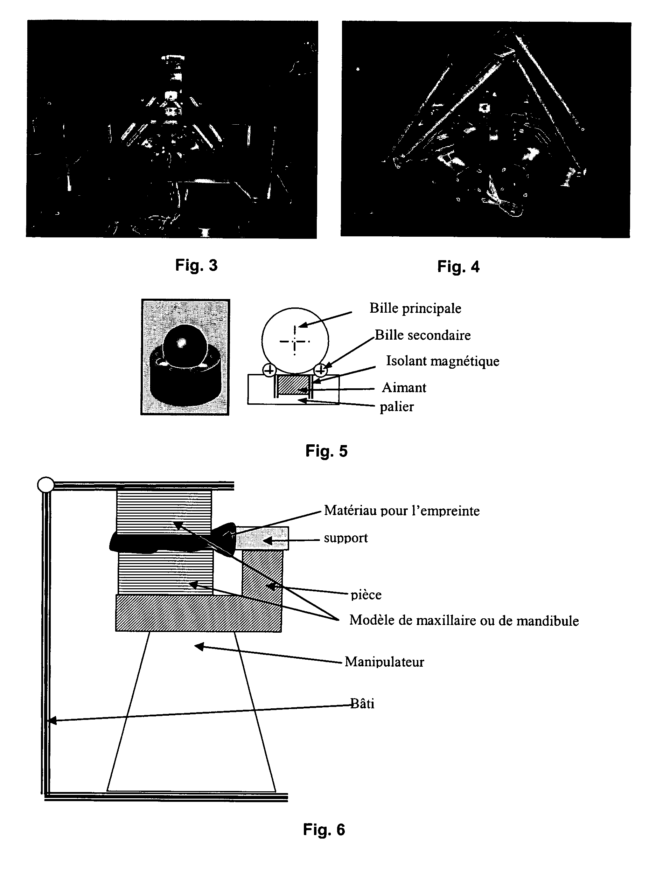

La présente invention concerne aussi un manipulateur qui est un dispositif permettant la mise en intercuspidation de modèles dentaires. Selon un mode particulier de l'invention, le dispositif est composé d'une base (plateau) fixe, d'une plate-forme mobile et de six « jambes ». La topologie (forme) du manipulateur est de type robotique parallèle i.e. chaque degré de liberté actionné se trouve en parallèle d'un autre contrairement à la robotique série. La topologie utilisée pour ce dispositif peut être de type plate-forme de Hunt. Les Figures 2 et 3 illustrent un manipulateur selon des modes de réalisation particuliers de l'invention.

Chaque jambe contient :
- une articulation rotoïde (1 degré de liberté en rotation) qui lie la jambe inférieure à la base,
- un segment rigide (jambe inférieure),
- une articulation sphérique (3 degrés de liberté en rotation),
- un segment rigide (jambe supérieure),
- une articulation sphérique (3 degrés de liberté en rotation) qui lie la jambe supérieure à la plate-forme. Les articulations sphériques ont été conçues spécialement pour l'application (voir ci-dessous) et permettent un grand débattement, pas de jeu et coût réduit. Un exemple de cette articulation sphérique est illustré à la Figure 5. La présente invention concerne donc aussi une articulation sphérique.

Du fait de sa topologie, un opérateur peut aisément déplacer la plate-forme à la main et garder sa capacité de « touché» pour trouver la position d'intercuspidation par exemple.

Le manipulateur comprend aussi des freins électromagnétiques sans jeu. Des capteurs de rotation sont également positionnés sur les articulations rotoïdes pour mesurer les variations de position.

Le manipulateur peut être connecté à un ordinateur avec une interface qui permet le calcul des positions de la structure. Le manipulateur est passif. Il est optimisé (taille des jambes, type de frein et de codeur, position des jambes sur la base et sur la plate-forme) pour être facilement manipulable à la main, pour être précis dans la mesure et sans jeu de la position. Le manipulateur selon l'invention permet aussi de bloquer avec précision et sans jeu la position et/ou de mesurer avec précision cette position

Le manipulateur de la présente invention peut être utilisé en chirurgie orthognathique comme un nouveau type d'articulateur. Il peut être utilisé lié à un logiciel de simulation pour permettre une interface à 6 degrés de liberté. De plus, il peut être utilisé en chirurgie, lorsqu'une position est trouvée manuellement par un chirurgien, lui permettre de la conserver (exemple d'utilisation comme casque stéréotaxique). Il peut aussi être utilisé dans le domaine de l'implant dentaire pour guider le fraisage d'un guide chirurgical en lien avec une simulation.

La présente invention concerne aussi une articulation sphérique adaptée pour le manipulateur de la présente invention. Les articulations sphériques selon l'invention sont basées sur un principe magnétique (Figure 5). Une bille principale (en matériau magnétique : bille de roulement) est attirée par un palier lisse à trois points de contact constitué chacun d'une bille (bille secondaire) de diamètre inférieur à la bille principale (en matériau magnétique : bille de roulement). Un aimant, dans un cylindre isolant (point de vue magnétique) est positionné dans le palier (en matériau magnétique) et permet d'attirer la bille principale et de la garder en contact avec les billes secondaires annulant toute possibilité de jeu.

L'articulation sphérique selon l'invention permet un lien mécanique à trois degrés de liberté en rotation avec les performances suivantes : pas de jeu, une grande précision (basé sur la sphéricité des billes de roulement), de grand débattement (approximativement 180° modulable selon les cas), un faible coût car basé sur des éléments commerciaux classique (billes de roulement et aimant), une grande précision (basée sur la sphéricité des billes de roulement) et un démontage aisé.

La présente invention concerne aussi un bâti permettant d'utiliser le manipulateur selon l'invention pour la chirurgie orthognathique. Un exemple de bâti est illustré à la Figure 6.

Le bâti selon l'invention permet la fixation des modèles en plâtre, le pressage de splint via une articulation rotoïde, le positionnement et / ou la fabrication du passage de repère et le positionnement du passage de repère.

En positionnant le passage de repère sur le bâti en relation avec le manipulateur, dans une position connue, on peut réaliser le passage de repère entre le logiciel de simulation (basé sur des images prises avec le même passage de repère) et le manipulateur.

Une pièce du bâti (Figure 6) permet également de fabriquer l'empreinte (mordu) du passage de repère d'une manière telle que le passage de repère entre le manipulateur et le logiciel de simulation soit simplifié. Ce procédé de fabrication comprend les étapes suivantes.
1. la pièce est montée sur le manipulateur.
2. on positionne l'empreinte de la mandibule (ou maxillaire) sur la pièce.
3. on positionne l'empreinte de la mandibule (ou maxillaire) sur le bâti,
4. on positionne les empreintes dentaires en position relative correcte grâce au manipulateur passif et grâce à un mordu en cire pris sur le patient,
5. on positionne le support du repère sur la pièce, et
6. on réalise la prise de l'empreinte dentaire du passage de repère dans cette position.

Ainsi l'information contenue dans le dispositif de passage de repère est plus grande car elle contient déjà une information de position relative entre la mandibule (ou le maxillaire) et la pièce. Il ne manque que la position relative maxillaire/mandibule dans le cas de la chirurgie orthognathique, ou aucune dans le cas d'implants dentaires. Ce procédé permet de gagner du temps et de la précision.

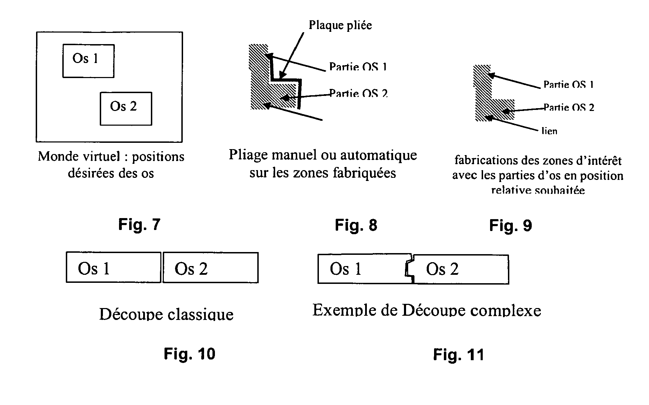

La présente invention concerne aussi une méthode (1) pour pré-plier tous types de plaques d'ostéosynthèse, conformément à un état virtuel (software) désiré du patient, qui une fois positionnée sur le patient réel, impose la même position relative des os que celle désiré. Cette méthode comprend la fabrication (par exemple par Prototypage rapide) des zones d'os (zones d'intérêts) en position relative souhaitée où seront positionnées les plaques et le prépliage des plaques. Les Figures 7 et 9 illustrent schématiquement un procédé de pré-pliage de plaques selon un mode de réalisation particulier de l'invention.

La Figure 7 illustre schématiquement la position désirée des os dans un monde virtuel (e.g. image 3D). La Figure 8 illustre schématiquement le pliage manuel ou automatique d'une plaque sur la zone d'os fabriquée. La Figure 9 illustre schématiquement une zone d'os fabriquée avec les parties d'os en position relative souhaitée (Figure 7).

Pour la fabrication des zones d'os il est possible d'utiliser une micro-imprimante 3D qui est un outil de prototypage rapide adapté aux petits gabarits, sur l'exemple de CT scan et micro-CT scan, ou PET scan et micro-PET scan.

La présente invention concerne également une méthode (2) pour obtenir la position des trous des plaques dans le logiciel sans connaître le modèle mécanique de la plaque (ce qui rend la méthode globale applicable a tous types de plaque). Cette méthode implique la mesure des positions de ces trous sur la plaque pliée par rapport au repère du bloc fabriqué, à transmettre dans le repère du logiciel de simulation.

La présente invention concerne aussi une méthode qui implique, outre les méthodes 1 et 2, la fabrication des mêmes zones avant déplacement des os et contenant une indication de la position du trait d'ostéotomie. Sur ces zones sont réalisés les mêmes trous que sur les zones où les plaques sont déformées. Sur ces zones sont fabriqués des guides chirurgicaux. Ceux-ci enregistrent les positions relatives des trous des plaques par rapport aux traits d'ostéotomies. Ces guides chirurgicaux peuvent être utilisés en salle d'opération pour marquer les positions des trous de la plaque à placer par rapport aux traits d'ostéotomies ou encore pour les positionner l'un par rapport à l'autre (trous par rapport aux ostéotomies) de manière précise indépendamment de l'utilisation d'un dispositif de navigation.

Les méthodes décrites ci-dessus sont particulièrement adaptée à la chirurgie orthognathique (fixation du maxillaire et/ou de la mandibule et/ou fragment de ceux-ci) conformément à une simulation, à la chirurgie maxillo-facial en général, en chirurgie orthopédique (redressement de vertèbre, pose de greffe,...) neurochirurgie, chirurgie otorhino-laryngologique ainsi que toutes disciplines médicales et vétérinaires utilisant des plaques d'ostéosynthèses.

La présente invention concerne aussi un système et un procédé semi actif de guidage Procédé semi actif de guidage intra opératoire d'un utilisateur caractérisé en ce qu'il comprend l'étape suivante : actionner un outil par exemple de marquage ou de découpe par l'intermédiaire d'une commande mixte comprenant :
un système de navigation conformément à une simulation (par exemple à la simulation obtenue par le procédé de simulation selon l'invention) et
l'ordre de l'utilisateur.

Selon le présent système, le chirurgien passe manuellement un outil capable de marquer la surface du tissu (durs ou mou). Un système de navigation mesure les positions relatives de cet outil et du tissu. Lorsque leur position relative est adéquate, le système envoie une impulsion qui actionne l'outil et marque automatiquement le tissu.

Le chirurgien réalise cela à divers endroits et peut ensuite utiliser ses outils classiques:
l'opération reprend son cours normal, il suit les marques effectuées comme guide.

Ce procédé semi-actif de guidage intra opératoire comprend les étapes suivantes :
actionner un outil d'un chirurgien par l'intermédiaire d'un système de navigation conformément à une simulation. Le chirurgien actionne également l'outil pour que l'ordre de mise en route s'effectue.

Seuls les outils de marquage du chirurgien sont guidés. Ainsi il n'est pas gêné par un quelconque fil ou field of view de la camera, système de repère encombrant sur le patient, etc, lorsqu'il effectue les tâches à risque telle que les découpes.

En chirurgie maxillo faciale, cet avantage est renforcé par le manque de place dans le champ opératoire. Ainsi au début d'opération, le chirurgien marque les positions des traits d'ostéotomies et des positions des trous des plaques et puis peut retirer le système de repérage ou de blocage pour reprendre un cours normal d'opération.

Ce principe peut s'appliquer à d'autres éléments. Par exemple, on peut équiper un outil de rabot de ce système (une fraise de chirurgien par exemple ou de laser en chirurgie laparoscopique pour brûler les tissus). Ainsi si le chirurgien doit réaliser une exérèse d'une partie de tissu, il lui sera possible de le faire avec précision (selon la forme exacte connue dans la simulation). L'outil (fraise, laser,...) sera actionné uniquement si la position est bonne. Ce qui permettra au chirurgien de « former » le tissu de la même manière que celle planifiée.

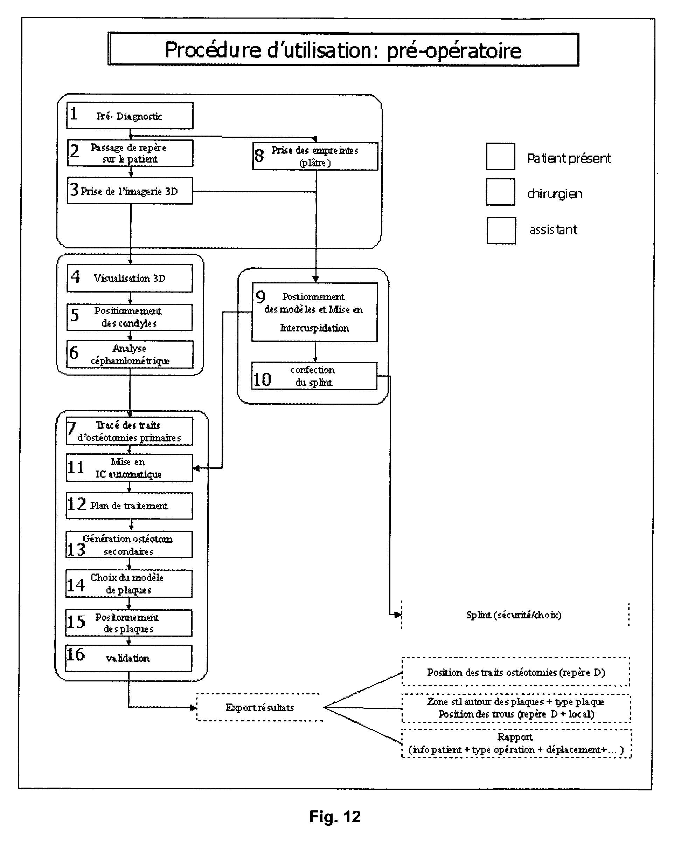

Ce procédé semi-actif de guidage peut être utilisé pour l'adaptation de greffe en salle d'opération, pour exérèse des os en chirurgie des os et pour la réalisation de découpes complexes dans les os pour obtenir une meilleure fixation. Les Figures 10 et 11 illustrent schématiquement un exemple de découpe classique (Figure 10) et un exemple de découpe complexe (Figure 11).

La présente invention permet l'intégration des informations 3D et 4D venant du CT Scan 3D, du manipulateur mécanique à 6 degrés de liberté, de la micro-imprimante 3D et de l'axiographie 4D en utilisant le passage de repère indépendamment des dents et des tissus mous de la face du patient.

Les exemples suivants sont donnés à titre illustratif et non limitatif de la présente invention, susceptible de variantes aisément accessibles à l'homme de l'art .

### Exemple 1

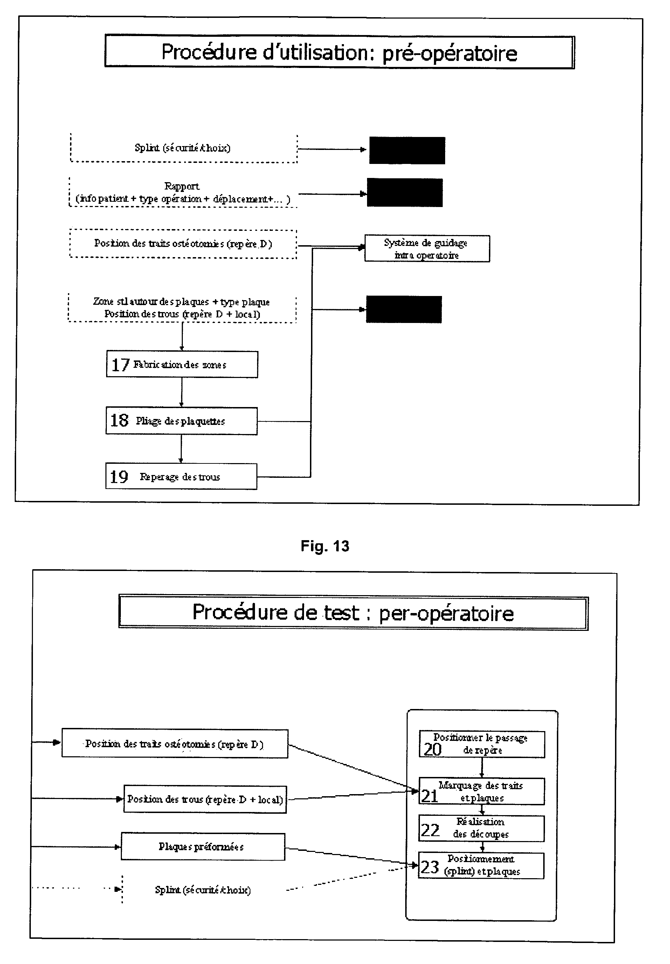

Un mode de réalisation particulier de l'invention est illustré aux Figures 12 et 13 ou les différentes étapes du procédé sont numérotées de 1 à 19.

Etape 1 : pré-diagnostic : Ce pré-diagnostic peut être effectué par un chirurgien pour décider l'utilisation de la procédure sur le patient.

Etape 2 : positionnement du passage de repère sur le patient : La fonctionnalité du passage de repère est double. (1) Le patient passe dans le dispositif d'imagerie avec ce système clairement visible pour le logiciel de simulation. Le logiciel détermine sur cette base dans quel repère doivent s'effectuer les déplacements et (2) permet également de repositionner les modèles en plâtre (ou autre matériau) de la dentition du patient dans une position relative similaire à celle de la dentition du patient lors de la prise de l'imagerie 3D et absolue par rapport au manipulateur selon l'invention.

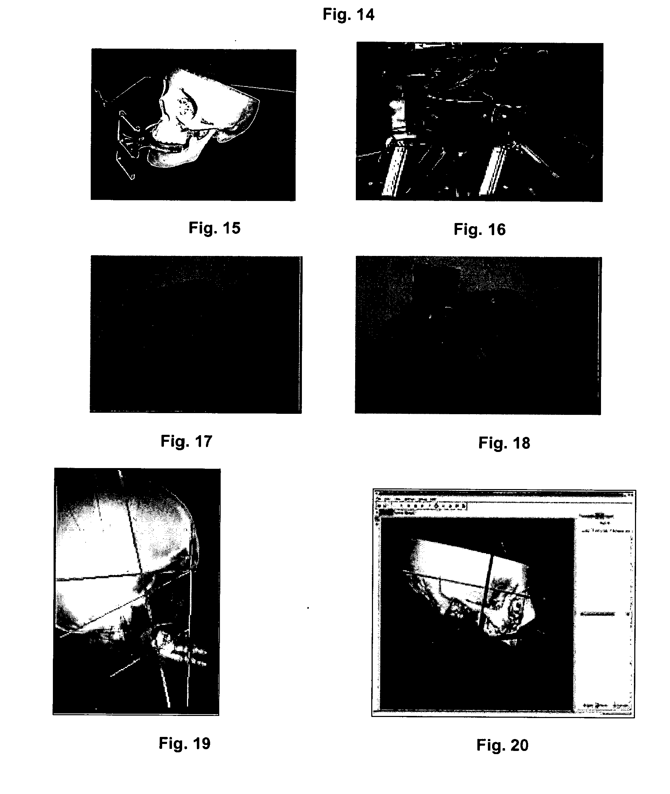

La Figure 1 illustre des exemples de passages de repères et la Figure 15 illustre le positionnement du passage de repère sur un patient représenté ici par un crâne.

Etape 3 prise de l'imagerie 3D : le patient et le passage de repère passent dans le système d'imagerie 3D, par exemple un CT scan, avec un protocole standard pour un scanner de la tête.

Etape 4 : visualisation 3D sur station de travail : Après un traitement d'image, le crâne du patient est visualisé sur l'écran de contrôle. Le passage de repère est détecté et mesuré.

Etape 5 : repositionnement des condyles : On permet à l'opérateur de repositionner, si nécessaire, les condyles du patient soit via des déplacements manuels ou automatiques sur le logiciel soit encore via une mesure directe sur le patient de son propre mouvement d'ouverture et fermeture de la mâchoire grâce à l'utilisation de l'axiographie 4D. Après cette étape, l'image du patient est prête pour réaliser l'analyse céphalométrique.

Etape 6 : analyse céphalométrique : l'analyse céphalométrique est réalisée en repérant différents points anatomiques précis sur la reconstruction 3D (modèle virtuel) du crâne du patient. De ces points sont déterminés des plans et différentes mesures qui permettent de définir, pour la première fois en réel 3D, les déplacements nécessaires à une bonne harmonie faciale. Dans le cas de la chirurgie orthognathique, cette analyse permet de déterminer la position optimale, sur le plant de l'harmonie faciale et non de l'intercuspidation, du maxillaire et de la mandibule.

Etape 7 : découpes primaires : Lors de cette étape, des découpes primaires peuvent être réalisées. En fonction des résultats de l'analyse céphalométrique, l'opérateur choisit le type d'opération nécessaire et réalise dès lors un trait de découpe virtuel dans le logiciel, mobilisant ainsi le maxillaire et/ou la mandibule ou une autre partie d'os. Lors de cette découpe l'opérateur peut être guidé, s'il le souhaite, par la visualisation des zones dangereuses à éviter : mise en évidence du nerf, des racines des dents, etc. Cette fonctionnalité peut être très utile pour son aspect pédagogique car ce sont des éléments qui pourront également être visualisés durant l'opération.

En parallèle une simulation sur le manipulateur est effectuée.

Etape 8 : prise des empreintes : Cette étape comprend la prise des modèles en plâtre (ou autre matériau) de la dentition par une méthode classique.

Etape 9 : mise en intercuspidation : cette étape comprend la mise en intercuspidation des modèles en plates sur le manipulateur. Les modèles en plâtre sont positionnés sur le manipulateur. Le montage se fait facilement par exemple par une vis de serrage. Au préalable, le passage de repère (le même que lors de la prise du CT scan) est positionné sur le manipulateur (Figure 16). Cela permet au dispositif de mesurer la position relative initiale des maxillaires du patient et de déterminer le système d'axe dans lequel travaille le logiciel de simulation. Ensuite l'opérateur manipule la mandibule en plâtre pour l'amener en intercuspidation. Lorsqu'il considère la position comme optimale, il appuie sur une pédale et déclenche le blocage de la position et la mesure précise du déplacement (6 degrés de liberté).

Etape 10 : confection du splint : Le maxillaire et la mandibule étant dans la position relative désirée, il est également possible au manipulateur de concevoir un splint. Cela n'est pas nécessaire dans la solution développée utilisant le pré-pliage de plaquettes.

Etape 11 : mise en intercuspidation automatique : A partir des données du manipulateur, une mise en intercuspidation automatique est effectuée sur le modèle virtuel. Dans le logiciel de simulation, le maxillaire ou la mandibule vont être déplacés par le vecteur déplacement transmis par le manipulateur, ce vecteur étant exprimé dans le repère commun transmis par le passage de repère. Ceux-ci vont donc se mettre en position d'intercuspidation maximale identique à celle déterminée sur le manipulateur par le chirurgien. Il faut noter que la procédure exacte à ce niveau peut varier et dépend du type d'opération : opération du maxillaire, opération de la mandibule, opération bi-maxillaire,....

D'autres types d'opérations sont possibles, par exemple des opérations fragmentaires qui consistent en la découpe d'un maxillaire en plusieurs fragments ; pour le bec de lièvre par exemple, et la procédure selon la présente invention sera toujours utilisable et performante.

Etape 12 : plan de traitement: Lors de l'étape suivante l'opérateur va combiner les résultats de l'analyse céphalométrique et de la mise en intercuspidation. Il va donc positionner le « bloc solidarisé» formé par le maxillaire et la mandibule, sur les plans dictés par l'analyse céphalométrique. On retrouvera ainsi une bonne fonction masticatoire et une bonne harmonie faciale. La procédure peut varier selon le type d'opération à simuler. Dans le cas d'une opération bi-maxillaire, il est possible de réaliser ce positionnement optimal. Par exemple, dans les opérations impliquant uniquement le maxillaire ou la mandibule, on ne réalisera que les déplacements pour obtenir l'intercuspidation.

Etape 13: génération des découpes secondaires sur le modèle virtuel. Le repositionnement du bloc formé par le maxillaire et la mandibule peut nécessiter une deuxième découpe de l'os du crâne (impaction ou rehaussement du maxillaire par exemple). Dans ce cas le programme calculera automatiquement ce deuxième trait de découpe pour qu'il soit en accord avec les déplacements.

Etape 14 : choix du type de plaque: Toutes les parties osseuses se trouvent dans les positions relatives souhaitées. Il est donc possible à partir de ce moment de choisir dans une bibliothèque le type de plaque de fixation (plaque d'ostéosynthèse) qui pourront être utilisées durant l'opération sur le patient. Il est aussi possible d'individualiser la forme de la plaque selon l'anatomie particulière du patient et selon les besoins de déplacement du maxillaire et ou de la mandibule (ou de tout autre fragment osseux). Une conception assistée de la plaque est généralisable à tout type de pathologie fracturaire humaine et non humaine (vétérinaire).

Etape 15 : choix des zones de positionnement des plaques : L'opérateur va ensuite positionner ces plaques là où il le désire sur le modèle virtuel du crâne du patient. Comme lors des découpes, des indications pourront apparaître pour guider ce positionnement.

Lorsque la position de la plaque est choisie et validée, la zone osseuse entourant les plaques sera isolée et sauvegardée (a un format informatique spécifique) pour être envoyée vers l'étape de fabrication des zones.

Etape 16 : validation : Lorsque toutes les plaques ont été placées virtuellement, l'opérateur valide la simulation et exporte les résultats vers les autres éléments de la procédure pour permettre le transfert de cette simulation en salle d'opération.

Partie pré-pliage des plaques : La zone sélectionnée pour le positionnement de la plaque a donc été séparée du reste du fichier. Cette zone va être concrétisé et fabriquée via l'utilisation de prototypage rapide.

Etape 17 : fabrication des zones : Dans cette étape, les zones sélectionnées pour le positionnement des plaques sont fabriquées, après traitement par reconstruction des triangles manquant sur l'image, via une méthode de prototypage rapide. La Figure 17 illustre une zone fabriquée par prototypage selon un mode de réalisation de l'invention. La méthode de prototypage peut être choisie parmi les méthodes de prototypage connues. Un exemple de méthode de prototypage consiste en la méthode nommée « z-corp ».

Par exemple, vu le petit gabarit des pièces à fabriquer, il est possible d'utiliser pour la fabrication des zones une micro-imprimante 3D.

Etape 18 : pré-pliage des plaques : Une fois les zones fabriquées, les plaques sont ensuite préformées. Dans cette étape, on reçoit le modèle de la zone sur laquelle va être placée et pliée la plaque originale non pliées choisie par l'opérateur. Sur ce modèle sont aussi visibles les zones dans lesquelles il ne peut placer de vis (racines dentaires, nerfs,...) ainsi que des informations de position de certains trous et de direction de la plaque. L'opérateur pourra alors soit utiliser des outils classiques pour positionner et déformer la plaque soit utiliser un système automatique de déformation. La Figure 18 illustre une zone fabriquée par prototypage comprenant les plaques préformées selon un mode de réalisation de l'invention.

Etape 19 : de repérage des trous : Ensuite, l'opérateur peut réaliser le pointage des trous de la plaque au moyen d'un système de navigation, en ayant au préalable passé le repère en repérant quelques points spécifiques. Cela permet au logiciel de connaître avec précision la position des trous de cette plaque sur le crâne et cela sans avoir besoin de son modèle où de sa géométrie complète.

Ces différentes étapes peuvent être réalisées autant de fois que le nombre de plaques utilisées pour cette opération.

Passage en salle d'opération :
Le procédé selon l'invention permet de fournir au chirurgien des plaques préformées ainsi que les zones fabriquées. La présente invention comprend aussi un procédé de chirurgie maxillo-faciale. Un mode de réalisation particulier de ce procédé est illustré à la Figure 14,
ou les différentes étapes sont numérotées de 20 à 23.

Etape 20 : Positionnements du repère : Au début de l'opération, après mise à nu de l'os maxillaire et ou mandibulaire selon les cas, on positionne et fixe les repères sur le patient. On réalise ensuite de brèves mesures de calibration telles que des mesures de points anatomiques etc.

Etape 21 : Repérage des découpes et plaques : Grâce au système de guidage selon l'invention, le chirurgien est guidé dans ses gestes et marque toutes les positions des trous des plaquettes et des traits d'ostéotomies.

Etape 22 : Réalisation des Découpes : Les lignes de découpes étant visibles pour le chirurgien grâce au procédé de simulation, il débute la découpe de l'os impliqué dans cette opération par exemple maxillaire et/ou mandibule. Il pourra selon les cas être guidé par la méthode de réalité augmentée pour l'orientation exacte de la découpe, pour l'orientation de direction pour des instruments chirurgicaux essentiels à l'opération (scie), pour avoir une indication visuelle de divers éléments de l'analyse céphalométrique 3D que sont les plans, points, droites, courbes, surfaces, volumes, les zones dangereuses à éviter pendant l'opération (par exemple : racines des dents, émergences de nerfs, vaisseaux).

Etape 23 : Fixation des découpes: Une fois l'élément i.e. maxillaire et/ou mandibule, suffisamment mobilisé, le chirurgien prend et positionne la plaque préformée pour cet endroit. Il lui suffit de placer les vis aux endroits marqués lors de l'étape de repérage décrite ci-dessus. Il réalise ensuite, selon les cas, le positionnement et la fixation des autres plaques pour cet élément.

Selon le type d'opération, bi-maxillaire par exemple, le chirurgien sera amené à effectuer la découpe d'un autre élément et recommencer l'étape de découpe et de fixation.

Bien que la présente invention ait été décrite dans le cadre de la chirurgie maxillo-faciale, le type de procédure selon l'invention peut être appliquée pour toutes opérations nécessitant la fixation (médicale, vétérinaire et non médicale) de tissus durs via tous types de plaque d'ostéosynthèse et indépendamment de leur modèle. L'utilisation de plaques d'ostéosynthèse selon la présente invention peut se faire sur humain ou sur animal.

Par exemple, la présente invention est applicable en cranio-maxillo-facial de manière générale, mais aussi pour tous types de fracture, pour le positionnement de greffe, pour le redressement de vertèbre, en orthopédie, en neurochirurgie, dans le domaine vétérinaire, etc.

Le procédé de simulation selon l'invention permet de ne plus passer par l'utilisation de splint en salle d'opération. La présente invention fourni des outils et éléments qui augmentent la précision du transfert des informations préopératoires vers la salle d'opération.

La présente invention comprend aussi un procédé de chirurgie maxillo-faciale utilisant les résultats du procédé de simulation selon l'invention. Ce procédé de chirurgie comprend l'utilisation de plaques pré-pliées sur des zones d'os, les dites zones d'os comprenant les informations spatiales d'orientation, de taille et de forme générées par le procédé de simulation selon l'invention.

### Exemple 2

Selon un mode de réalisation particulier du procédé selon l'invention, l'analyse céphalométrique peut être réalisée de la façon décrite ci-dessous, en utilisant une analyse ACRO 3D [OLSZEWSKI R. et al. Computer Assisted Radiology and Surgery, In CARS 2003. LEMKE HU. et al. (Eds), London, Elsevier 2003 ;1235-1240 (1432 pages)].

L'analyse ACRO 3D regroupe deux théories différentes du développement de l'extrémité céphalique. Il s'agit d'abord du concept d'équilibre idéal et individuel, décrit par Delaire [DELAIRE J. Rev Stomatol 1978 ;79 :1-33.], qui doit exister entre les diverses pièces osseuses du squelette cranio-facial dans la dimension sagittale. Cet état d'équilibre est représenté par une construction 2D de diverses lignes de référence, tracées sur une téléradiographie de profil (Figure 19), et présentant l'alignement idéal et individuel (pas de norme statistique) des pièces osseuses que devrait posséder le patient. L'analyse ACRO 3D utilise l'extrapolation 3D de l'analyse 2D de Delaire. Ceci permet d'obtenir la visualisation de deux plans fondamentaux de référence : le plan occlusal idéal individuel, responsable de la symétrie cranio-caudale cranio-faciale, ainsi que le plan antérieur d'équilibre de la face, responsable de la symétrie antéro-postérieure cranio-faciale.

L'extrapolation 3D transforme la notion des lignes d'équilibre en 2D (Figure 19) en une notion de plans de symétrie en 3D (Figure 20).

L'analyse ACRO 3D propose trois plans sagittaux médians pour les différents compartiments du complexe cranio-maxillo-facial: plan sagittal basi-crânien, orbito-maxillaire et mandibulaire. Ces plans ne correspondent pas à un idéal, ils sont tributaires de la pathologie sous jacente. Le réalignement éventuel de ces plans entre eux permet de symétriser le squelette du patient. Ainsi, on généralise l'approche des matrices fonctionnelles aux autres paires de nerfs crâniens et des structures volumiques, qui participent aussi à l'élaboration 3D de la face. Enfin, au-delà de son utilisation en chirurgie maxillo-faciale thérapeutique, l'analyse ACRO 3D permet une description volumétrique de la voûte du crâne et des diverses cavités du massif cranio-facial, ce qui est essentiel à une reconstruction 3D réaliste au départ de crânes partiellement conservés en médecine légale.

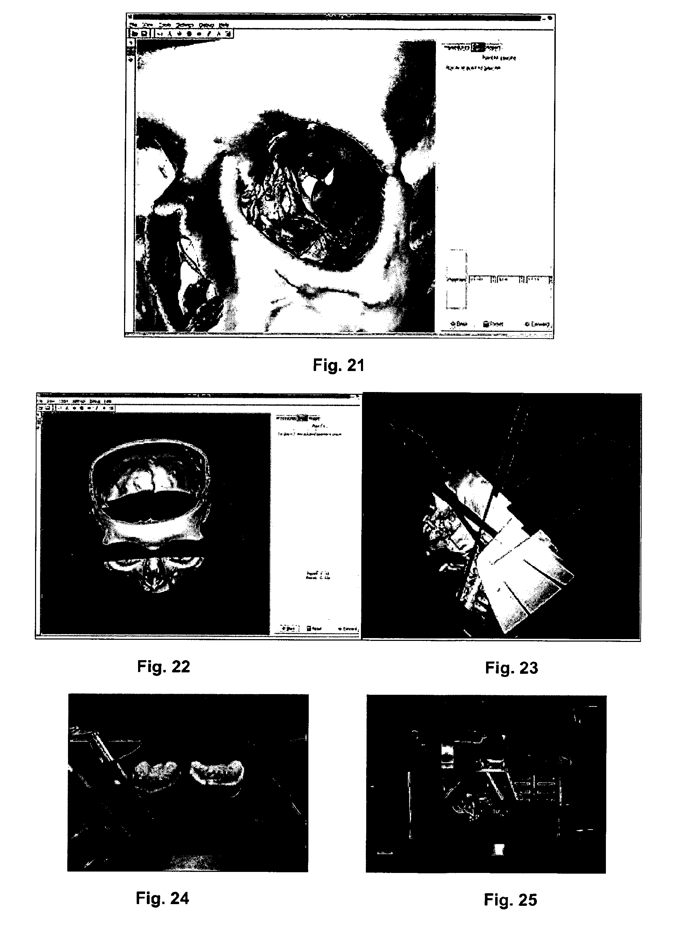

Pratiquement, le patient doit passer un examen tomodensitométrique du vertex crânien au menton (épaisseur de coupe : 1 mm, coupes jointives, pitch : 0,7, vitesse du tube RX : 1 rotation/s, FOV : 210mm, 120 KV, 200 mAs). Les images sont stockées sur CD Rom, lues et reconstruites en 3D dans le logiciel ACRO 3D, développé en étroite collaboration interdisciplinaire avec les ingénieurs de traitement de signal. Le logiciel permet, grâce aux divers outils incorporés, d'accéder rapidement au diagnostic et à la prise de décision thérapeutique en 3D. Le logiciel ACRO 3D fonctionne sous système d'exploitation Windows. Il réalise des reconstructions 3D au départ des images 2D (format Dicom) du CT scan. On peut réaliser des segmentations semi-automatiques de divers types de tissus. Le placement des points de référence se fait aussi bien sur les images 2D que sur les reconstructions 3D (Figure 21).

La synchronisation des vues 2D et 3D permet de visualiser en même temps les plans en 3D et les lignes en 2D. Tout objet ajouté sur les reconstructions 3D (point, sphère, ligne, courbe, plan, volume) peut être déplacé. On peut mesurer des distances sur les images 2D et les reconstructions 3D. On peut mesurer les angles en 2D et 3D, les surfaces en 2D et les volumes en 3D. Il est possible de mesurer le point milieu d'une surface courbe en 3D. Des dessins surajoutés sont possibles sur toutes les surfaces des reconstructions 3D. Grâce à l'utilisation conjointe des boutons gauche (rotation) - roulette (translation) - droit (zoom avant arrière) de la souris, il est possible de naviguer au sein du massif cranio-maxillo-facial. Cette navigation augmente la précision du placement des points de référence et de mesures directement sur les reconstructions 3D. Des bibliothèques de cas cliniques sont prévues pour aider le chirurgien dans le placement des points de référence et pour avoir des vues d'ensemble des diverses pathologies cranio-maxillo-faciales en 3D.

Par défaut, le logiciel propose de débuter l'analyse ACRO 3D, cependant tout utilisateur désirant mettre en place sa propre analyse céphalométrique 3D est libre de la réaliser grâce à ces divers outils de base.

L'approche modulaire (11 modules) de l'analyse ACRO 3D permet de compartimenter l'étude 3D de la pathologie cranio-maxillo-faciale et de la rendre plus simple et plus accessible aux conclusions diagnostiques et thérapeutiques. Au départ de trois plans crâniens (C1- plan supérieur de la base du crâne, C2- frontière cranio-faciale, C3-pente basilaire) on définit la position idéale individuelle du maxillaire et mandibule par rapport à la base du crâne (Figure 22).

Avec deux plans crâniens (C2- frontière cranio-faciale, C4-hauteur du crâne) et l'étude volumique, on décrit les rapports entre la base du crâne et la voûte crânienne (Figure 23). Les trois plans sagittaux médians (plan sagittal basi-crânien, orbito-maxillaire et mandibulaire) analysent l'(as)symétrie du patient. Le plan antérieur d'harmonie de la face (F1) détermine la position 3D antéro - postérieure de la face par rapport à la base du crâne. Deux plans faciaux déterminent la position idéale des branches montantes de la mandibule par rapport à la base du crâne et au maxillaire (F2-plan moyen de l'harmonie de la face, F3-plan postérieur de l'harmonie de la face). Deux plans faciaux (F4-hauteur faciale théorique, F5-plan cervico-palatin) déterminent les hauteurs théoriques des étages maxillaire et mandibulaires en rapport avec les déterminants basicrâniens et cervicales.

Le plan d'harmonie antéro - postérieur (F6) définit la position idéale des angles mandibulaires par rapport à la base du crâne et au maxillaire. Le plan basilaire mandibulaire (F7) facilite le diagnostic des hyper- et hypo- développements des branches horizontales.

Le plan occlusal idéal individuel (F8) est fondamental pour le diagnostic et traitement 3D des hyper - et hypo - divergences maxillo-faciales.

Après chaque module d'analyse ACRO 3D, contenant deux à trois plans, un rapport automatique est établi, avec les mesures des distances, des angles et des volumes correspondants.

En fin d'analyse ACRO 3D, on procède à l'établissement du plan de traitement en fonction du diagnostic établi. On désigne les pièces osseuses à déplacer (maxillaire et/ou mandibule) et on quantifie l'importance et la direction 3D du déplacement à réaliser.

Pour les utilisateurs demandant uniquement un diagnostic rapide, il existe un outil de tendances. Celui-ci permet de visualiser, sous forme d'échelle de couleur, un renforcement d'une tendance vers une catégorie de pathologie cranio-maxillo-faciale (pro- ou rétro- maxillie ou mandibulie, hyper- ou hypo- divergence maxillaire ou mandibulaire) à partir de divers indices de l'analyse céphalométrique 3D.

### Exemple 3 :

Les principales étapes de la procédure d'utilisation du manipulateur d'intercuspidation selon un mode de réalisation particulier de l'invention sont illustrées aux Figures 24 à 27.

Un exemple de manipulateur selon l'invention est représenté à la Figure 3. Le manipulateur a une structure parallèle à 6 degrés de liberté. Elle permet d'être manipulée aisément à la main, de mesurer la position de sa plate-forme et de bloquer sa position.

Les empreintes en plâtre à l'état brut sont représentées à la Figure 24. Dans la procédure selon l'invention, il n'est pas nécessaire de préparer les empreintes en retirant le plâtre superflu.

La Figure 25 montre les empreintes en plâtre fixées sur le manipulateur. Le manipulateur est constitué du système mécanique et d'un bâti permettant la fixation du maxillaire.

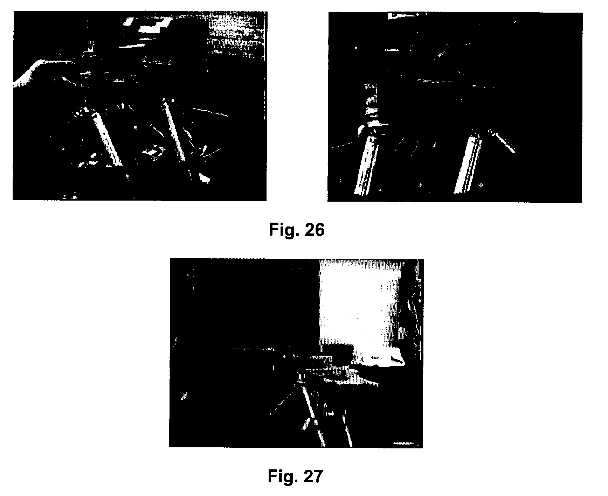

La Figure 26 illustre le positionnement du repère sur les empreintes positionnées sur le manipulateur. Pour terminer cette étape de positionnement, la structure doit être remontée pour mettre en contact le repère et le maxillaire. A noter que si l'opérateur revient sur sa décision, il ne doit pas refaire toute la procédure ni reprendre des empreintes en plâtre comme c'est le cas dans la procédure actuelle.

La Figure 27 montre l'opérateur qui amène la mandibule en intercuspidation. L'opérateur manipule alors la mandibule et l'amène en intercuspidation. Lorsque la position lui semble bonne, il bloque la position de la structure via une pédale. S'il le souhaite il peut alors confectionner un splint de manière classique.

La présente invention permet de simuler les interventions orthognathiques en préopératoire, d'aider un chirurgien à positionner, aussi correctement que possible, des fragments ostéotomisés ou fracturés, d'accroître très sensiblement la précision finale, de diminuer le risque de complications et d'échecs opératoires et de diminuer le temps nécessaire à la phase préparatoire. Elle peut être appliquée à la chirurgie orthopédique, neurochirurgie, chirurgie ORL, radiologie interventionnelle etc.

## Revendications

1. Procédé de simulation médicale, **caractérisé en ce qu'**il comprend les étapes suivantes (a) l'affichage d'une image 3D du crâne d'un patient sur un écran, ledit crâne comprenant un passage de repère, (b) la mise en intercuspidation d'empreintes dentaires dudit patient après y avoir positionné le passage de repère décrit à l'étape (a), (c) la translation de la mise en intercuspidation de l'étape (b) à l'image 3 D , (d) l'analyse céphalométrique de l'image 3 D de l'étape (a), (e) l'établissement d'un plan de traitement et (f) la génération à partir de l'étape (e) de plaque d'ostéosynthèse pré-pliée.

2. Procédé de simulation de chirurgie maxillo-faciale **caractérisé en ce qu'**il comprend les étapes suivantes :
i) le positionnement d'un passage de repère sur les dents d'un patient et le passage du patient dans un système d'imagerie 3D pour générer un modèle virtuel du crâne du patient,
ii) l'analyse céphalométrique 3D du modèle virtuel obtenu à l'étape (i),
iii) l'obtention d'empreintes dentaires dudit patient et leur positionnement dans un dispositif de déplacement manuel et de mise en intercuspidation desdites empreintes dentaires,
iv) le positionnement du passage de repère de l'étape (i) sur les empreintes dentaires et la mise en intercuspidation des empreintes à l'aide du dispositif de déplacement et de mise en intercuspidation,
v) la mesure des positions des empreintes après mise en intercuspidation et la transmission desdites positions sur le modèle virtuel généré à l'étape (i),
vi) la détermination sur le modèle virtuel d'une ou plusieurs zones de positionnement pour des plaques d'ostéosynthèses, et le prépliage virtuel desdites plaques.

3. Procédé selon la revendication 2, **caractérisé en ce que** les étapes (ii) et (iii) se font en parallèle.

4. Procédé selon la revendication 2, **caractérisé en ce que** l'étape (iii) a lieu avant l'étape (ii).

5. Procédé selon la revendication 2 ou 3, **caractérisé en ce que** lorsqu'une position optimale d'intercuspidation est obtenue à l'étape (ii) la position est bloquée et le déplacement mesuré et l'impact affiché.

6. Procédé selon l'une quelconque des revendications 2 à 5, **caractérisé en ce que** ledit procédé comprend une étape de fabrication de zones de positionnement déterminées à l'étape (vi).

7. Procédé selon la revendication 6, **caractérisé en ce que** ladite fabrication se fait par prototypage.

8. Procédé selon la revendication 6 ou 7, **caractérisé en ce que** ledit procédé comprend une étape de pré-pliage de plaques d'ostéosynthèses à l'aide desdites zones de positionnement.

9. Procédé selon l'une quelconque des revendications précédentes, **caractérisé en ce qu'**il comprend après l'analyse céphalométrique une étape de tracé de traits d'ostéotomies virtuels.

10. Procédé selon l'une quelconque des revendications précédentes, **caractérisé en ce qu'**il comprend entre l'étape (a) et (b), ou (i) et (iii) une étape de repositionnement des condyles du patient sur le modèle virtuel.

11. Procédé selon l'une quelconque des revendications 2 à 11, **caractérisé en ce que** dispositif de déplacement manuel et de mise en intercuspidation desdites empreintes dentaires est le dispositif tel que définit à l'une quelconque des revendications 13 à 20.

12. Procédé semi actif de guidage intra opératoire d'un utilisateur **caractérisé en ce qu'**il comprend l'étape suivante : actionner un outil par exemple de marquage ou de découpe par l'intermédiaire d'une commande mixte comprenant :
un système de navigation conformément à la simulation obtenue par le procédé selon l'une quelconque des revendications 1 à 10 et
l'ordre de l'utilisateur.

13. Dispositif adapté au déplacement manuel et à la mise en intercuspidation d'empreintes **caractérisé en ce qu'**il comprend : une base fixe sur laquelle sont connectées six jambes articulées, et l'extrémité supérieure de chacune des six jambes est connecté à une plateforme mobile.

14. Dispositif selon la revendication 12, **caractérisé en ce que** chaque jambe comprend:
- une articulation rotoïde à 1 degré de liberté en rotation qui lie un premier segment rigide à ladite base,
- le premier segment rigide,
- une articulation sphérique qui lie le premier segment à un second segment rigide,
- un second segment rigide, et
- une articulation sphérique qui lie le second segment rigide à ladite plate-forme.

15. Dispositif selon la revendication 13, **caractérisé en ce que** l'articulation sphérique à 3 degrés de liberté en rotation.

16. Dispositif selon la revendication 13 ou 14, **caractérisé en ce que** l'articulation sphérique comprend une première bille positionnée sur un palier lisse à trois points de contact constitué chacun d'une bille secondaire de diamètre inférieur à la première bille, ledit palier comprenant un aimant dans un cylindre isolant positionné dans le palier.

17. Dispositif selon la revendication 15, **caractérisé en ce que** ladite première bille, les billes secondaires et le palier sont en matériau magnétique.

18. Dispositif selon l'une quelconque des revendication 13 à 16, **caractérisé en ce que** le dispositif comprend de plus un ou plusieurs freins électromagnétiques sans jeu liés à ladite base.

19. Dispositif selon l'une quelconque des revendication 13 à 17, **caractérisé en ce que** le dispositif comprend de plus un ou plusieurs capteurs de rotation positionnés sur lesdites articulations rotoïdes pour mesurer les variations de position.

20. Dispositif selon l'une quelconque des revendication 13 à 18, **caractérisé en ce que** le dispositif est connecté à un ordinateur avec une interface qui permet le calcul des positions du dispositif.

21. Articulation sphérique **caractérisée en ce qu'**elle comprend une première bille positionnée sur un palier lisse à trois points de contact constitué chacun d'une bille secondaire de diamètre inférieur à la première bille, ledit palier comprenant un aimant dans un cylindre isolant positionné dans le palier.

22. Articulation sphérique selon la revendication 20, **caractérisé en ce que** ladite première bille, les billes secondaires et le palier sont en matériau magnétique.
